# EUROPÄISCHE PATENTANMELDUNG

(11) **EP 0 818 542 A1**
(43) Veröffentlichungstag der Anmeldung: **14.01.1998**
(21) Anmeldenummer: 97110681.0
(22) Anmeldetag: 01.07.1997
(51) Int. Cl.: C12Q 1/68

(54) **Verfahren zur Stabilisierung von Nukleinsäuren vor deren Isolierung aus Blutproben**

(30) Priorität: 09.07.1996 AT 407/96
(71) Anmelder: Labordiagnostika Gesellschaft mbH, 1110 Wien (AT)
(72) Erfinder: Kury, Friedrich, Mag., 1180 Wien (AT)
(74) Vertreter: Puchberger, Rolf, Dipl. Ing.

(57) **Zusammenfassung**

Es wird ein Verfahren zur Stabilisierung von Nukleinsäuren in einer Blutprobe und zur Isolierung von Nukleinsäuren aus einer Blutprobe beschrieben, bei welchem das Blut unmittelbar nach der Abnahme vom Patienten mit Guanidiniumthiocyanat gemischt wird, so daß die gesamten in der Blutprobe enthaltenen Nukleinsäuren während der Weiterverarbeitung vollständig erhalten bleiben.

## Beschreibung

Die Erfindung betrifft ein Verfahren zur Stabilisierung von Nukleinsäuren vor deren Isolierung aus Blutproben.
Virale Infektionen können einerseits auf dem klassischen Weg über den Nachweis von viralen Proteinen oder Antikörpern gegen die Viren und andererseits durch den Nachweis der den Viren entsprechenden Nukleinsäuren diagnostiziert werden.

Bei der zuletzt genannten Methode muß man zwischen DNA Viren und RNA Viren, zu denen unter andere HIV und das Hepatitis C Virus gehören, unterscheiden.

Insbesondere beim Nachweis von RNA Viren besteht aufgrund der geringen Stabilität der nachzuweisenden Nukleinsäuren die Gefahr, daß diese Nukleinsäuren enzymatisch angebaut werden, wenn die Blutprobe nicht rasch in einem Labor weiterverarbeitet wird. Oft ist der Ort, an welche dem Patienten Blut abgenommen wird nicht ident mit dem Ort, an dem das Blut analysiert wird. Bisher wurde daher aus dem Blut durch Zentrifugieren Serum prepariert und dieses Serum für Transport und Aufbewahrung eingefroren. Nach dem Auftauen wurden dann die Nukleinsäuren nach Standardprotokollen extrahiert, wobei die Viruspartikel aus dem Serum durch Zentrifugieren vor der Extraktion angereichert werden können. Die Nukleinsäuren werden dann z.B. mittels Polymerase-Ketten-Reaktion ("PCR") nachgewiesen.

Ein Nachteil dieses bekannten Verfahrens ist, daß bei der Serumgewinnung die Gefahr besteht, daß der Virus RNA angebaut wird und somit nicht mehr nachgewiesen werden kann. Insbesondere eine quantitative Bestimmung, welche auch für die Diagnostik große Bedeutung hat, ist unmöglich. Weiters besteht keine Möglichkeit, in Blutzellen integrierte Viren nachzuweisen und/oder sonstige RNA Spezies aus Blutzellen zu isolieren (z.B. für die Tumor-Früherkennung), da, wie oben beschrieben, nur das Serum zur Analyse verwendet wird. Ein weiterer Nachteil ist, daß die kannten Standardprotokolle zur Isolierung von RNA viele Manipulationen erfordern, womit einerseits das Risiko für das Personal erhöht wird, andererseits die Gefahr einer Verschleppung von Probenmaterial zwischen den Proben steigt. Da die Nachweismethoden jedoch sehr sensitiv sind, können schon geringste verschleppte Volumina einer positiven Probe andere Proben falsch positiv werden lassen.

Aufgabe der vorliegenden Erfindung ist es, ein Verfahren zur Stabilisierung von Nukleinsäuren vor deren Isolierung aus Blutproben zu finden, bei dem die zuvor genannten Nachteile nicht auftreten.

Die Aufgabe wird erfindungsgemäß dadurch gelöst, daß die Blutprobe unmittelbar nach der Abnahme vom Patienten mit Guanidiniumsalz, z.B. Guanidiniumthiocyanat, vermischt wird und infolge die Nukleinsäuren aus Blutzellen sowie von in Blutzellen oder im Serum enthaltenen Viren nach an sich bekannten Verfahren isoliert werden.

Die Weiterverarbeitung kann z.B. mit folgenden Schritten erfolgen:
a) Isolierung von DNA:
   - Mischen der Blut-Guanidiniumsalz-Lösung mit Äthanol
   - Abtrennen des aus dem vorigen Schritt resultierenden, DNA-hältigen Niederschlages durch Zentrifugieren
   - Waschen des Niederschlages mit Äthanol und Auflösen des Niederschlages in wäßriger Lösung
b) Isolierung von RNA:
   - Mischen der Blut-Guanidiniumsalz-Lösung mit einem Gemisch aus Phenol und einer wäßrigen Guanidiniumsalz-Lösung
   - Zugabe von Chloroform
   - Trennen in eine organische, proteinhältige Phase und eine wäßrige, RNA-hältige Phase durch Zentrifugieren
   - Versetzen der wäßrigen Phase mit Isopropanol
   - Abtrennen des aus dem vorigen Schritt resultierenden, RNA-hältigen Niederschlages durch Zentrifugieren
   - Waschen des Nierschlages mit Äthanol und Auflösen des Niederschlages in wäßriger Lösung

Erfindunsgemäß liegt das Guanidiniumsalz in Pulverform vor, wodurch im Vergleich mit einer Guanidiniumsalz-Lösung die höhere Stabilität des Guanidiniumsalzes genutzt wird. Vorzugsweise ist das Guanidiniumsalz Bereits in dem an sich bekannten zur Blutabnahme bestimmten System, z.B. in einer Eprouvette mit Unterdruck vorhanden und wird darin mit dem Blut vermischt, bzw. auch darin aufgelöst. Das erfindungsgemäße bevorzugte Mischungsverhältnis ist 4 Millimol Guanidiniumsalz pro Milliliter Blut. Das Guanidiniumsalz lysiert Blutzellen und Viruspartikel und denaturiert allgemein Proteine. Somit werden insbesonders auch spezielle Nukleinsäure abbauende Enzyme (RNasen und DNasen) desaktiviert.

Je weniger Verfahrensschritte bzw. Pipettierschritte zur Isolierung der Nukleinsäuren notwendig sind, desto geringer ist das gesundheitliche Risiko für das Laborpersonal und desto geringer ist auch das Risiko einer Verschleppung von Probenmaterial. Ist das Guanidiniumsalz bereits im zur Blutabnahme bestimmten Gefäß enthalten (geschlossenes System), bedeutet dies einen Schritt weniger und somit erhöhte Sicherheit. Das erfindungsgemäße Verfahren ist gegenüber bisher kannten Verfahren sicherer, schneller, einfacher und ermöglicht dabei sowohl die Isolierung von Nukleinsäuren der in Blutzellen enthaltenen Viren als auch von Nukleinsäuren von im Serum enthaltenen Viren, wobei die Stabilisierung aller Nukleinsäuren gewährleistet ist.

Um das erfindungsgemäße Verfahren noch deutlicher darzustellen, wird nachfolgend ein konkretes Ausführungsbeispiel bechrieben.

In ein evakuiertes Blutabnahme-Röhrchen mit 0,94 g Guanidiniumthiocyanat in Pulverform werden 2 ml Blut abgenommen. Alternativ dazu kann mit einer herkömmlichen Spritze Vollblut abgenommen und 2 ml davon in einen Behälter mit 0,94 g Guanidiniumthiocyanat transferiert werden. Durch Schütteln des geschlossenen Behälters löst sich das Guanidiniumthiocyanat im Blut auf. Das Guanidiniumthiocyanat bewirkt, daß Blutzellen und Viruspartikel lysiert werden und Proteine denaturiert werden. Somit werden auch Nukleasen desaktiviert. In diesem stabilisierten Zustand kann die Blut-Guanidiniumthiocyanat-Lösung bei Raumtemperatur transportiert oder bis zu 24 Stunden gelagert werden. Zur längeren Lagerung ist eine Kühlung auf -20°C notwendig.

Zur Isolierung der DNA werden 500 µl der Blut-Guanidiniumthiocyanat-Lösung mit 500 µl 4 M Guanidiniumthiocyanat-Lösung, gepuffert mit 0,1 M Tris/Cl, pH 8, gemischt. Durch Zugabe von 500 µl 99%-igem Äthanol wird DNA präzipitiert und durch Zentrifugieren bei 15.000xg für 5 min pelletiert. Nach zweimaligem Waschen des Niederschlages mit 95%-igem Äthanol wird der DNA-hältige Niederschlag in wäßrigem Puffer oder in Wasser gelöst.

Zur Isolierung der RNA werden 3 ml eines Gemisches aus Phenol und einer wäßrigen Guanidiniumthiocyanat-Lösung, im Verhältnis 3 Teile Phenol, gesättigt und gepuffert mit 0,1 M Tris/Cl, pH 8, und 1 Teil 4 M Guanidiniumthiocyanat-Lösung in Aqua dest., der Blut-Guanidiniumthiocyanat-Lösung beigement.

Dann werden 1,2 ml von dem entstehenden Blut-Guanidiniumthiocanat-Pnenol-Gemisch mit 120 µl Chloroform und 60 µl Natriumacetat-Lösung gemischt. Beim anschließenden Zentrifugieren, 15 min mit 15.000xg bei 4°C, trennen sich die beiden entstandenen Phasen und man erhält eine organische, proteinhältige Phase, dann eine Interphase, welche denaturierte Proteine und DNA enthält und oben eine wäßrige, RNA-hältige Phase.

Von der oberen, wäßrigen, RNA-hältigen Phase werden 600 µl Isopropanol gemischt. Dann folgt eine Zentrifugation mit 15.000xg für 15 min bei 4°C, wodurch die RNA präzipitiert wird.

Es folgt ein dreimaliges Waschen des Niederschlages, indem der Isopropanol-Überstand abgegossen wird und 600 µl 70%-iger Äthanol zum Niederschlag zugegeben wird. Durch Aufschütteln des Niederschlages und Zentrifugieren bei 15.000xg für 5 min wird dieser von Guanidiniumsalzen gewaschen. Abschließend wird der Äthanolüberstand vollständig entfernt und der RNA-hältige Niederschlag in einem wäßrigen Puffer oder in Wasser gelöst.

## Patentansprüche

1. Verfahren zur Stabilisierung von Nukleinsäuren in einer Blutprobe, dadurch gekennzeichnet, daß die Blutprobe unmittelbar nach der Abnahme vom Patienten mit Guanidiniumsalz, z.B. Guanidiniumthiocyanat, vermischt wird und infolge die Nukleinsäuren aus Blutzellen sowie von in Blutzellen oder im Serum enthaltenen Viren nach an sich bekannten Verfahren isoliert werden.

2. Verfahren nach Anspruch 1, dadurch gekennzeichnet, daß das Guanidiniumsalz in Form von Pulver vorliegt und durch Schütteln im Blut aufgelöst wird.

3. Verfahren nach einem der vorhergehenden Ansprüche, dadurch gekennzeichnet, daß das Guanidiniumsalz bereits in dem an sich bekannten, zur Blutabnahme bestimmten System, z.B. in einer Eprouvette mit Unterdruck, vorliegt.

4. Verfahren nach einem der vorhergehenden Ansprüche, dadurch gekennzeichnet, daß pro Milliliter Blut vorzugsweise 4 Millimol Guanidiniumsalz vorbanden sind.
